# EUROPEAN PATENT APPLICATION

(11) **EP 4 163 024 A1**
(43) Date of publication of application: **12.04.2023**
(21) Application number: 22386070.1
(22) Date of filing: 26.10.2022
(51) Int. Cl.: B08B 15/00, F24F 11/77, F24F 8/108, B08B 7/00

(54) **MODULAR DEVICE FOR GAS SUCTION AND PURIFICATION FROM ENCLOSED SPACES**

(30) Priority: 30.09.2021 GR 20210100651
(71) Applicant: Giallourakis, Georgios-Hans, 15669 Papagos (Attikis) (GR); Giallouraki, Alkmini-Nelly, 15669 Papagos (Attikis) (GR); Neamonitos, Konstantinos, 12461 Haidari (Attikis) (GR)
(72) Inventor: Giallourakis, Georgios-Hans, 15669 Papagos (Attikis) (GR); Giallouraki, Alkmini-Nelly, 15669 Papagos (Attikis) (GR); Neamonitos, Konstantinos, 12461 Haidari (Attikis) (GR)
(74) Representative: Yazitzoglou, Evagelia S.

(57) **Abstract**

A modular device for gas suction and purification from enclosed spaces is presented comprising two separate main chambers, wherein the first main chamber (1) is the main gas entry chamber and the second main chamber (11) is the main chamber of their purification, wherein the first main chamber (1) is rectangular parallelepiped-shaped with a first large anterior surface (2) through which forced gas entry takes place and a second large posterior surface (5) through which gas exit through outlet piping (6) connected to a central pipe (7) by means of microprocessor-adjusted speed suction pump (8) takes place, wherein within the first main chamber (1) are placed many electronically microprocessor-adjusted speed electric fans (3), small-sized, of low power and little weight, which cover almost the entire surface area of the first large anterior surface (2) and of which the blades move forward the gases into the first main chamber (1).

## Description

The present invention relates to a modular device for gas suction and purification from enclosed spaces according to claim 1 as well as a large surface area gas suction and purification system that may be deployed and cover very large surfaces for the purification of indoor air from smokes, odors or even infectious droplets.

Such gases are generated e.g. during laser hair removal, electric welding or sick people infectious droplet exhalation, as well as in conditions of smoke or toxic gas generation in business premises or gatherings. The device according to the invention is suitable for use in venues without good air-conditioning, with stagnant air and humidity. Concerning laser use during hair removal, it has the following distinctive characteristic: to avoid creation of a burn mark on the skin during contact with the beam, a very cold air flow is injected in the operation area however, this results in a high dispersion of the burned hair odor in the space. Accordingly it isn't possible to confine the microbial load created by the exhalation of the patient moving in healthcare settings such as medical practice or hospitals or ICUs where the air must be as sterile as possible, free from airborne pathogens carried by medical practitioners and nurses; the same applies to cinemas, restaurants etc. where a dispersion of airborne viruses such as flu, COVID, etc. probably takes place.

Devices for gas suction and purification from enclosed spaces are known from the state of the art. Gas suction from an enclosed space is usually done by means of piping in the beginning of which is adapted a hood whereas at their end and in general outside the enclosed space is connected a pump. Said pump creates vacuum pressure at the end of the installation thereby gas suction takes place.

The vacuum pressure created at the point of the pump is zero atm in ideal conditions. Taking into consideration the aerodynamic frictions from the pump up to the hood, where the gas entry takes place, one can clearly see that the system loses some of its gas suction capacity. Furthermore, after the placement of the hood in the space its position can't be changed, at least not until the rearrangement of space itself. Likewise addition of hoods requires space rearrangement as well as change of pump power.

In any case, increase of pump power usually demands as well a gas duct diameter increase, something not possible in spaces of limited height, such as offices.

The present invention intends to present a modular device for gas suction and purification from enclosed spaces without the drawbacks of the state of art as well as large surface area gas suction and purification system that may be deployed and cover very large surfaces for the purification of indoor air from smokes, odors or even infectious droplets.

This is achieved by means of the modular device for gas suction and purification from enclosed spaces according to claim 1. A gas suction and purification system is presented in claim 6. Further implementations and applications of the invention is the object of the dependent claims.

According to the invention a modular device for gas suction and purification from enclosed spaces is proposed comprising two separate main chambers, wherein the first main chamber is the main gas entry chamber and the second main chamber is the main chamber of their purification, wherein the first main chamber is rectangular parallelepiped-shaped preferably with a depth less than 20% of the other two dimensions, has a first large anterior surface through which forced gas entry takes place and a second large posterior surface through which gas exit through outlet piping connected to a central pipe by means of microprocessor-adjusted speed suction pump takes place.

According to the invention within the first main chamber are placed many electronically microprocessor-adjusted speed electric fans, small-sized, preferably with a surface ranging from 60 to 150 cm2, of low power, preferably between 2 and 10 Watt and of little weight, preferably between 100 and 300 grams which cover almost the entire surface area of the large anterior first main chamber surface and of which the blades move forward the gases into the first main chamber.

The second main chamber of the device is connected to the first main chamber through the central pipe, which comprises a microprocessor-controlled outlet valve of the gases into the environment without their passing through the second main chamber, wherein the microprocessor is connected to a first sensor for monitoring the pressure within the first main chamber and to a second sensor located after the suction pump within the central pipe and adjusts the suction pump speed so that the pressure of the first main chamber is always lower than that of the outlet piping, so that the first main chamber can receive new compressed air volume with the least possible power consumption without the fans having to run at full speed.

The second main chamber may comprise a rectangular parallelepiped, of which the anterior side has a slot for the first main chamber gas entry whereas the posterior side has second slot, through which the gases exit after purification, whereas a lateral side of the parallelepiped opens in such a way that the necessary purification filters can be placed slidingly in a parallel manner and sequentially, which filters are positioned into sliding frames, which don't allow side gas leak from one sliding frame to the other.

Furthermore, the modular device for gas suction and purification from enclosed spaces is connectable concerning control and operation to similar such units to create a large surface area gas suction and purification system. Such a system comprises two or more gas suction and purification devices, interconnected concerning control and operation, wherein each gas suction and purification device comprises a person sensor which monitors the proximity of the position of the persons in the space with respect to the device, wherein a microprocessor based on the sensor data increases or reduces the speed of each system fan or of the fans of each device.

The difference of the present invention compared to the state of the art lies in the fact that the gases are pushed into the device by means of positive pressure, namely compression. Thus there's no restraint concerning pressure increase. Positive pressure is guaranteed by the fans placed on the anterior large first main chamber surface and guiding by force the gases into the first chamber in combination with the suction pump located within the central pipe after the exit from the first chamber; this way the cross section of the device piping does not have to be large as the gases are moved forward into it with a pressure higher than that of the atmosphere.

In contrast to the already known gas suction and purification techniques, with the present invention it is possible to create a system using a large number of uniform main chambers of little weight over very large surfaces, each one having a customized suction adjustment and changing position easily, given that each device has its own piping arrangement. Such an arrangement is unachievable for extractor hoods.

The present invention will be more clearly understood from the following description based on a certain example.

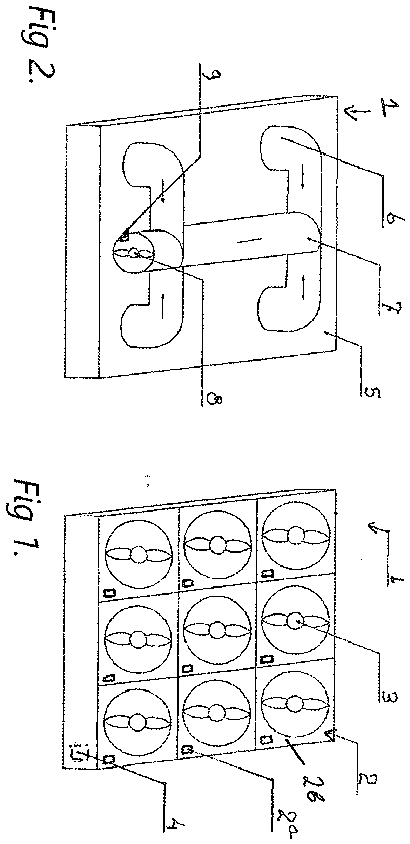

The operation principle is presented in Figures 1 - 3. In these figures the principles of the technical design are not followed faithfully. Illustrations are made without many details and are simplified.

In figure 1 the large anterior surface 2 of the first main chamber 1 is depicted, which is rectangular parallelepiped-shaped preferably with a depth less than 20% of the other two dimensions. Indicative dimensions are 60 cm^{∗}60 cm ^{∗}12 cm. Through this surface forced gas entry takes place, whereas gas exit takes place through a second large posterior surface 5 depicted in figure 2 through outlet piping 6 connected to a central pipe 7 by means of microprocessor-adjusted speed suction pump 8.

In the first main chamber 1 the gases are guided and compressed by a system of many adjusted fans 3 of little weight, small size and low power demand, the fans being placed in base frame 2β all over the anterior surface 2 of the first main chamber 1. In base-frame 2β are installed the power cords of each fan 3 which terminate in socket 2α on the base-frame, so that replacement of each fan 3 is done directly. Then the gases are suctioned and abducted from the first main chamber 1 into the second large posterior surface 5 of chamber 1. The inlet fans 3 are of adjusted speed by means of the microprocessor so that optimal suction with the least power consumption, lowest noise level and least intervention in the general volume of the conditioned air is possible, so that a large fresh air volume entry is not required.

According to the invention the microprocessor is connected to sensor 4 for monitoring the pressure within the main chamber 1 seen in figure 1 and to second sensor 9, located after the adjustable suction pump 8, within the central pipe 7 seen in figure 2, and adjusts the suction pump 8 speed, so that the pressure of the first main chamber 1 is always lower than that of the outlet piping 6 so that the first main chamber 1 can receive new compressed air volume with the least possible power consumption without the fans 3 having to run at full speed. Such a differential pressure system is unachievable with the previous hood techniques. The small adjustable inlet fans 3 will move forward the gases into chamber 1 continuously and smoothly without creating pressure saturation within it. As the first main chamber 1 has all over its anterior surface the small adjustable fans 3 it can be of little depth relative to its dimensions, have low power motors with small impellers generating lesser noise, fewer shocks and, most importantly, be of little weight in contrast to the previous hood techniques, of which the pumps are adapted at the end of the piping and preferably outside the building, as they are extremely heavy.

Operation of hood is based on vacuum pressure creation. The maximum that such a suction pump can achieve by means of hood is almost zero atm. The other main difference of the device of the invention compared to hood is the use of a large number of small pumps in contrast to other techniques using pumps as big as possible, which however increases the noise. The device, which is subject to the laws of fluid mechanics has to follow the respective principles for its best performance. The external form and dimensions of the device are standardized in module form. This enables many devices to interconnect forming a system and to form very large surfaces, while the depth remains the same as that of the initial device.

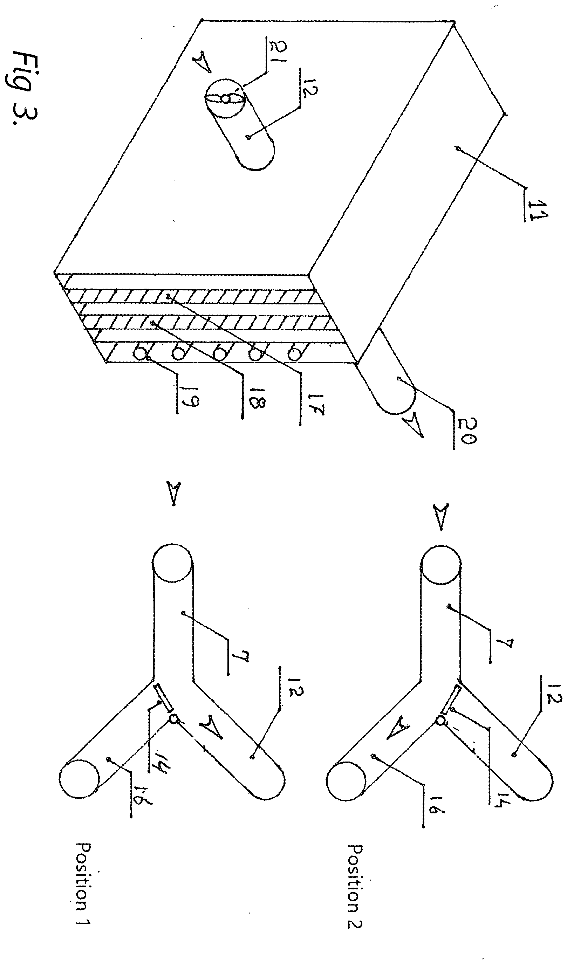

The gases can be trapped in the first main chamber 1 of the device and then suctioned out of the first main chamber 1 by means of the adjusted speed suction pump 8 which will release them outside through piping; however, if these gases can't be securely discarded due to polluted environment or conditioned air temperature maintenance in the space, they are processed in the second main chamber 11 of the device presented in figure 3, through the central pipe 7, a microprocessor-controlled outlet valve 14 of the gases and a pipe 12 connected to inlet slot of the second main chamber 11, when valve 14 is in position 1. When valve 14 is in position 2 the gases are released into the environment through outlet valve 14 of the gases and a pipe 16 without passing through the second main chamber 11. Alternatively the microprocessor-controlled outlet valve 14 may be substituted by manual deflector (moveable wall) mounted on hinge.

As seen in figure 3 the second main chamber 11 comprises a rectangular parallelepiped of which the anterior side has slot, through which the gases of the first chamber 1 enter, through pipe 12 whereas on the posterior side has second slot through which the gases exit through pipe 20. A lateral side of the parallelepiped, apart from the two abovementioned, opens in such a way that the necessary filters can be placed slidingly in a parallel manner and sequentially, positioned into frames, which do not allow side gas leak from one sliding frame to the other. These filters are sequentially: activated carbon filter 17 for odors, HEPA filter 18 for suspended dust microparticles and viruses, whereas in the rest of the chamber 11 UV lamps 19 are disposed for sterilization.

If gas flow due to frictions in the piping and second main chamber 11 is not enough then there is made provision for second microprocessor-adjusted speed adjusted suction pump 21 which is activated at the inlet of the second main chamber 11. The second main chamber must render the air after the processing close to the floor at selected points so that they don't leave still gas (mass) spots which will probably be polluted. Another space where the present device is applied is in rooms in which air and sunlight enter through one side only. In such a space, devices installed on the opposite wall achieve direct and very fast refreshing of the entire air mass, as the air entering through the windows of the one wall will exit by means of the devices through the opposite wall/side. The same method may be applied at any point in a space, where still air spots/nests appear. Another application example is the warehouses having only one point of air entry, namely the door/entrance of the site. In these spaces there is usually high humidity as well as musty odor. The device can refresh the air totally when placed in the appropriate position even without use of large ventilation piping cross-sections, as the gases are pushed outside the space by positive pressure.

The reason for which the first main chamber 1 and the second main chamber 11 do not form an integral part lies in the fact that the first chamber 1 must weigh as little as possible as one or more first main chambers 1 are preferably positioned on the ceiling or the walls of each space. For example they can cover almost entire room ceilings or walls. In the case where a big part of the ceiling is covered the infected droplets will travel a smaller distance from their point of origin, namely the head of the patient, to their suction point, in particular upwards, reducing by far the likelihood of virus dispersion to neighboring healthy persons in the space.

This possibility of placing the aspiration devices on the ceiling makes this method innovative compared to other ones. In venues with standardized false ceiling such as business premises, the dimension of the main device may be standardized indicatively to 60cm^{∗}60cm^{∗}12cm depth, namely the dimension of the standard false ceiling panel. The first main chambers of the devices can be placed on the false ceiling as the weight of each one is similar to that of the respective neon luminaires placed on false ceilings. Moreover, all of the piping and wiring of each device may pass through the clearance between false ceiling and ceiling, creating a product that combines perfectly aesthetics with functionality.

In addition, in case of a system consisting of at least 2 devices, after customized adjustment of the fan speed of each device, it is possible to select a different suction rate per point in the venue, thereby achieving better and fast targeted gas removal as well as energy saving and noise minimization. Photocells placed on the face of each device monitor the proximity of the position of persons in the space relative to a certain device and increase or reduce the fan speed accordingly. This process is possible by means of microprocessor, which monitors/estimates how close the person is to each device so that it increases the speed of all or some of the fans of the proximate one to achieve the best possible result.

The customized suction rate of each space may be monitored with the use of a special non-toxic smoke agent, so that visualization of gas flow and the whole process, as well as easy adjustment of the optimal fan rotation speed may be achieved. The cost of non-toxic smoke generation machines is minimal. Such smoke use is made in wind tunnel laboratories studying gas fluid mechanics. This way of adjustment significantly increases the effectiveness and energy efficiency of the invention.

## Claims

1. Modular device for gas suction and purification from enclosed spaces comprising two separate main chambers, wherein the first main chamber (1) is the main gas entry chamber and the second main chamber (11) is the main chamber of their purification, wherein the first main chamber (1) is rectangular parallelepiped-shaped with a first large anterior surface (2) through which forced gas entry takes place and a second large posterior surface (5) through which gas exit through outlet piping (6) connected to a central pipe (7) by means of microprocessor-adjusted speed suction pump (8) takes place, wherein within the first main chamber (1) are placed many electronically microprocessor-adjusted speed electric fans (3), small-sized, of low power and little weight, which cover almost the entire surface area of the first large anterior surface (2) and of which the blades move forward the gases into the first main chamber (1), wherein the second main chamber (11) is connected to the first main chamber (1) through the central pipe (7), a microprocessor-controlled outlet valve (14) of the gases or a manual deflector in a first position and a pipe (12) connected to inlet slot of the second main chamber (11), whereas when the outlet valve (14) or the manual deflector are in a second position the gases are released into the environment without passing through the second main chamber (11), wherein the microprocessor is connected to a first sensor (4) for monitoring the pressure within the first main chamber (1) and to a second sensor (9) located after the suction pump (8) within the central pipe (7) and adjusts the suction pump (8) speed so that the pressure of the first main chamber (1) is always lower than that of the outlet piping (6) so that the first main chamber (1) can receive new compressed air volume with the least possible power consumption without the fans having to run at full speed.

2. Modular device for gas suction and purification from enclosed spaces according to claim 1, **characterized in that** the second main chamber (11) comprises a rectangular parallelepiped of which the anterior side has slot for the first main chamber (2) gas entry through pipe (12) whereas on the posterior side it has second slot through which exit the gases through pipe (20) after purification, while a lateral side of the parallelepiped opens in such a way that the necessary purification filters are placed slidingly in a parallel manner and sequentially, positioned into sliding frames, which don't allow side gas leak from one sliding frame to the other, whereas the purification filters are sequentially activated carbon filter (17) for odors, HEPA filter (18) for suspended dust microparticles and viruses whereas in the rest of the chamber (11) UV lamps (19) are disposed for sterilization.

3. Modular device for gas suction and purification from enclosed spaces according to claim 1 or 2, **characterized in that** if the gas flow due to frictions in the piping and second main chamber (11) is not enough then there is made provision for second microprocessor-adjusted speed adjusted suction pump (21) which is activated at the inlet of the second main chamber (11).

4. Modular device for gas suction and purification from enclosed spaces according to claim 1, 2 or 3, **characterized in that** the depth of the first main chamber (1) is less than 20% of the other two dimensions.

5. Modular device for gas suction and purification from enclosed spaces according to claim 1, 2 or 3, **characterized in that** it is connectable concerning control and operation to such similar units in such a way that a large surface area gas suction and purification system is created.

6. Large surface area gas suction and purification system, **characterized in that** it comprises two or more gas suction and purification devices according to claim 5, interconnected concerning control and operation, wherein each gas suction and purification device comprises a sensor for the persons in the space, which monitors the proximity of their position in the space with respect to the device, wherein a microprocessor based on the sensor data increases or reduces the speed of each system fan or of the fans of each device.
